Europäisches Patentamt

European Patent Office

Office européen des brevets

(19)

(11) Veröffentlichungsnummer: **0 000 519**
**B1**

(12) **EUROPÄISCHE PATENTSCHRIFT**

(21) Anmeldenummer: **78100394.2**

(22) Anmeldetag: **14.07.78**

(51) Int. Cl³: **C 07 C 109/04**

(54) Verfahren zur Herstellung von 2,2'-Dichlorhydrazobenzol

(30) Priorität: **27.07.77 DE 2733747**

(43) Veröffentlichungstag der Anmeldung:
**07.02.79 Patentblatt 79/03**

(45) Bekanntmachung des Hinweises auf die Patenterteilung:
**23.07.80 Patentblatt 80/15**

(84) Benannte Vertragsstaaten:
**BE CH DE FR GB NL**

(56) Entgegenhaltungen:
**US - 2 794 046**
**US - 3 156 724**

(73) Patentinhaber: **Hoechst Aktiengesellschaft**
**Postfach 80 03 20**
**D - 6230 Frankfurt**
**Main 80 (DE)**

(72) Erfinder: **Planker, Siegfried, Dr.**
**Kastanienweg 17**
**D - 6240 Königstein Taunus (DE)**
**Baessler, Konrad, Dr.**
**Drosselweg 1**
**D - 6000 Frankfurt**
**Main (DE)**
**Fuchs, Otto, Dr.**
**Oestricher Weg 11**
**D - 6000 Frankfurt**
**Main (DE)**

Courier Press, Leamington Spa, England.

## Verfahren zur Herstellung von 2,2'-Dichlorhydrazobenzol

Die Erfindung betrifft ein Verfahren zur Herstellung von 2,2'-Dichlorhydrazobenzol durch katalytische Reduktion von o-Nitrochlorbenzol mit Wasserstoff.

Aus der US—PS 3.156.724 ist bekannt, 2,2'-Dichlorhydrazobenzol durch katalytische Hydrierung von o-Nitrochlorbenzol herzustellen. Der Hydrierkontakt besteht aus Palladium oder Platin. Als Reaktionsmedium dient eine 2 bis 20 %ige wäßrige Lösung von Natrium- oder Kaliumhydroxid, insbesondere 13 bis 14 %ige Natronlauge, gegebenenfalls unter Zusatz eines organischen Lösemittels, vorzugsweise eines nicht wassermischbaren aromatischen Kohlenwasserstoffs wie Benzol, Toluol oder Xylol. Die Temperatur liegt zwischen 40—100°C, vorzugsweise bei 60 bis 70°C, der Wasserstoff(über)druck bei etwa 0,4 bis 7,8 bar (20—125 psi,abs.), vorzugsweise etwa 0,75 bis 1,8 bar (25—40 psi,abs.). Für die Bildung von 2,2'-Dichlorhydrazobenzol werden dem Reaktionsgemisch Zusätze von Naphthalinderivaten, wie Naphthochinon-(1,4) oder 2,3-Dichlor-naphthochinon-(1,4) zugegeben. Die so erhaltenen Ausbeuten an 2,2'-Dichlorhydrazobenzol variieren zwischen 80 und 90 %, die Chlorabspaltung soll gering sein.

Wie eine Nacharbeitung ergab, fallen bei einem erneuten Einsatz der Edelmetallkatalysatoren von Ansatz zu Ansatz nicht nur die Ausbeuten ab, sondern gleichzeitig steigen die Reaktionszeiten bei sinkender Aktivität der Edelmetallkatalysatoren. Beides ist für eine wirtschaftliche Durchführung der Reduktion unerwünscht. Die Chlorabspaltung liegt mit 7 bis 8 % schon beim ersten Einsatz recht hoch.

Es wurde nun überraschend gefunden, daß die katalytische Reduktion von o-Nitrochlorbenzol zu 2,2'-Dichlorhydrazobenzol mit Wasserstoff in wäßriger Natron- oder Kalilauge, insbesondere einer 10 bis 25 gewichtsprozentigen Natronlauge, und in Gegenwart eines nicht wassermischbaren aromatischen Lösemittels, insbesondere eines Kohlenwasserstoffs wie Benzol, Toluol oder Xylol, mit Edelmetallkatalysatoren, vorzugsweise Palladium-, Platin- oder modifizierten beispielsweise sulfidierten (gemäß DE—PS 1.959.578), insbesondere sulfitierten Platin-auf-Kolenstoff-Katalysatoren (gemäß DE—PS 2.105.780) bei einem Wasserstoff(über)druck von 1 bis etwa 10, vorzugsweise bis 6 bar und einer Reduktionstemperatur von etwa 50 bis 80, insbesondere 60°C zu hohen und gut reproduzierbaren Ausbeuten führt, wenn als Co-Katalysatoren Derivate des Anthrachinons, vorzugsweise Hydroxyanthrachinone, z.B. β-Hydroxyanthrachinon oder 2,6-Dihydroxyanthrachinon, zugesetzt werden.

Von besonderem Vorteil ist, daß die Edelmetallkatalysatoren bei Verwendung der Anthrachinonderivate sehr oft zurückgeführt werden können, ohne daß sie einen Aktivitätsabfall erleiden. Selbst nach z.B. zehnmaligem Einsatz der Edelmetallkatalysatoren werden konstante Ausbeuten in der gleichen Reduktionszeit wie beim Startansatz erhalten.

Die Anthrachinonderivate beschleunigen die Reduktion der einzelnen Reaktionsstufen, insbesondere der Azoxy- und Azostufe, wesentlich stärker als Naphthochinonverbindungen, so daß eine niedrigere Temperatur während der gesamten Reaktionszeit ermöglicht wird und dabei noch kürzere Reaktionszeiten als bei Einsatz der bekannten Naphthochinone erzielt werden.

Zusätzlich erfolgt eine wesentlich geringere Chlorabspaltung: sie beträgt bei Verwendung von Palladium 4 %, bei unmodifiziertem Platin weniger als 2 % und bei sulfitiertem Platin (hergestellt nach DE—PS 2.105.780) unter 1 %. Überraschend war, daß der sulfitierte Platinkatalysator, der bisher nur zur katalytischen Reduktion von halogenhaltigen Nitroaromaten zu den entsrechenden Aminen in neutralem oder schwach saurem Medium geeignet erschien, auch zur Reduktion von o-Nitrochlorbenzol zu 2,2'-Dichlorhydrazobenzol in stark alkalischer Lösung eingesetzt werden kann.

Ein weiterer Vorteil ist, daß z.B. das β-Hydroxyanthrachinon nach der Reduktion aus der wäßrigen Mutterlauge durch Einstellen eines pH von 3 bis 4 praktisch quantitativ fällbar und ohne Reinigung mehrmals wieder einsatzfähig ist, während das 2-Hydroxy-3-chlornaphthochinon-(1,4) (entstanden während der Reduktion aus 2,3-Dichlornaphthochinon-(1,4)) durch eine aufwendige Abwasserreinigung beseitigt werden muß.

Die Einsatzmenge an Anthrachinonen ist gering, sie liegt unter denen der Naphthochinonderivate. So ist z.B. ein Gewichtsverhältnis β-Hydroxyanthrachinon zu o-Chlornitrobenzol von 0,003 bis 0,008, insbesondere 0,004 : 1 ausreichend, um auch das als Zwischenstufe auftretende Dichlorazoxybenzol über das Dichlorazobenzol gleichmäßig bis zur Hydrazoverbindung durchzureduzieren, während vom 2,3-Dichlornaphthochinon-(1,4) die doppelte Menge notwendig ist, um wenigstens beim ersten Einsatz der Edelmetallkatalysatoren vergleichbare Ergebnisse zu erzielen.

Für die Wirtschaftlichkeit des Verfahrens ist von Bedeutung, daß der Edelmetallkatalysator — bei zuverlässiger Reproduzierbarkeit der Ausbeuten und Produkteigenschaften auch nach vielmaligem Einsatz — nur in einem Gewichtsverhältnis von Nitroverbindung zu Platin oder Palladium zwischen etwa 4000 : 1 und 1500 : 1, vorzugsweise 2500 : 1, eingesetzt zu werden braucht.

Als Reaktionsmedium verwendet man eine 16 bis 25 %ige Natronlauge in der Menge, daß nach Reaktionsende durch das entstandene Reaktionswasser eine 10 bis 15 %ige Natronlaugekonzentration entsteht.

Auch hier zeigen die Anthrachinone gegenüber den Naphthochinonen Vorteile. Während mit Naphthochinon die besten Ergebnisse mit einer 16 %igen Natronlauge in einem Gewichtsverhältnis o-Nitrochlorbenzol zu NaOH (100 %) wie 1 : 0,095 erzielt werden, erlauben die Anthrachinone eine Erhöhung der NaOH-Konzentration bis auf 25 % und einen geringeren Einsatz von Natronlauge in einem Gewichtsverhältnis von o-Nitrochlorbenzol zu NaOH (100 %) wie 1 : 0,071, ohne daß dabei die Reaktionsgeschwindigkeit verlangsamt wird. Der Einsatz einer ca. 25 %igen NaOH bedeutet gegenüber einer 16 %igen NaOH nach obigen Gewichtsverhältnissen eine Verbesserung der Raumausbeute von ca. 20 %.

Die Reaktionstemperatur liegt bevorzugt zwischen 55 bis 60°C, der Wasserstoffdruck bevorzugt zwischen 1 bis 6 bar, wobei es vorteilhaft ist, den Druck während der Reduktion innerhalb der angegebenen Grenzwerte langsam steigen zu lassen.

Bei dem erfindungsgemäßen Verfahren wird die Reduktion von o-Nitrochlorbenzol zu 2,2'-Dichlorohydrazobenzol unter Verwendung eines mit Wasser nicht mischbaren Lösemittels, wie z.B. Benzol, Toluol, Xylol, Äthylbenzol oder deren technische Gemische, beispielsweise das unter dem Namen "Solventnaphtha" handelsübliche Gemisch aus m-Xylol und Äthylbenzol, durchgeführt.

Die Reduktion wird besonders vorteilhaft so vorgenommen, daß man o-Nitrochlorbenzol, wäßrige Natronlauge, das Anthrachinon-Derivat, z.B. $\beta$-Hydroxyanthrachinon, Lösemittel, ein Emulgiermittel und einen Edelmetallkatalysator in einen üblichen Autoklaven einfüllt und nach Verdrängen der Luft mit Stickstoff unter Rühren aufheizt. Man ersetzt den Stickstoff durch Wasserstoff und drückt so lange Wasserstoff auf, bis kein Druckabfall mehr erfolgt. Die gewünschte Reaktionstemperatur wird durch Kühlung oder Heizung von außen aufrechterhalten.

Nach Beendigung der Reduktion wird unter Stickstoff der Katalysator abfiltriert und ohne Reinigung in die nächste Reduktionscharge zurückgeführt, wobei er wenigstens zehnmal eingesetzt werden kann.

Zur Bestimmung der Ausbeute wird nach Abtrennung der Wasserphase von der Lösemittelphase, in der das gebildete 2,2'-Dichlorhydrazobenzol und o-Chloranilin gelöst sind, mit verdünnter Salzsäure das o-Chloranilin ausgewaschen, das Lösemittel abdestilliert und die Hydrazoverbindung getrocknet. Da das Produkt in ausreichender Reinheit anfällt, kann auch die organische Phase direkt der Umlagerung mit Mineralsäuren zu 3,3'-Dichlorbenzidin zugeführt werden.

Das erfindungsgemäße Verfahren gestattet es also, 2,2'-Dichlorhydrazobenzol in besonders wirtschaftlicher Weise durch katalytische Reduktion von o-Nitrochlorbenzol in Gegenwart von Anthrachinonen in hohen Ausbeuten und gut reproduzierbar herzustellen. Die Vorteile des erfindungsgemäßen Verfahrens seien an folgenden Beispielen näher erläutert. Die Prozentangaben beziehen sich hierbei auf das Gewicht, sofern nichts anderes angegeben ist.

### BEISPIEL 1

In einem 2 1-Stahlautoklaven mit Magnethubrührung, Heizvorrichtung und Kühlung werden vorgelegt:

| | | |
|---|---|---|
| 630 | g | o-Nitrochlorbenzol (4 Mol), |
| 200 | ml | "Solventnaphtha", |
| 180 | g | 25 %ige Natronlauge, |
| 2,5 | g | $\beta$-Hydroxyanthrachinon, |
| 2 | g | Emulgator (handelsübliches Emulgatorgemisch, bestehend im wesentlichen aus Natrium-dodecylbenzolsulfonat mit geringen Anteilen an Ölsäure, dem Natriumsalz der $C_{13}$—$C_{15}$-Alkylsulfamidocarbonsäure und schwach chlorierten, langkettigen Kohlenwasserstoffen in wäßrigem iso-Butanol), |
| 0,25 | g | Paaladium, in Form von 5 g 5 % Palladium-auf-Kohlenstoff-Katalysator. |

Nach Verdrängen der Luft im verschlossenen Autoklaven mit Stickstoff wird das Reaktionsgemisch unter Rühren auf 60°C erwärmt und Wasserstoff bis auf 3 bar aufgedrückt. Entsprechend der Wasserstoffaufnahme wird der Wasserstoffdruck bis zum Ende der Reduktion bis auf 6 bar gesteigert. Die Reduktion ist beendet, wenn die Aufnahme des Wasserstoffs abbricht, was nach 5 Stunden der Fall ist. Nach Reaktionsende wird die Reaktionsmischung auf 80°C erhitzt und bei dieser Temperatur der Palladium-Kohle-Katalysator abfiltriert. Das Filtrat wird mit 600 ml "Solventnaphtha" verdünnt und die organische Phase, die das 2,2'-Dichlorhydrazobenzol sowie das als Nebenprodukt gebildete o-Chloranilin enthält, von der wäßrigen Phase getrennt.

Zur Bestimmung der Ausbeute wird in üblicher Weise das o-Chloranilin durch zweimaliges Waschen mit 5 %iger Salzsäure herausgelöst und das "Solventnaphtha" im Vakuum entfernt.

Die Ausbeute beträgt 84 % d. Th. an 2,2'-Dichlorhydrazobenzol vom Schmelzpunkt 85—86°C sowie 10 % d. Th. an o-Choranilin, jeweils bezogen auf das eingesetzte o-Nitrochorbenzol.

Der abfiltrierte Palladium-auf-Kohle-Katalysator wird ohne Reinigung noch mindestens zehnmal eingesetzt und die Reduktion in gleicher Weise durchgeführt. Bei allen Folgeansätzen wird, ohne Minderung der Qualität, die gleiche Ausbeute an 2,2'-Dichlorhydrazobenzol wie beim Startansatz erhalten. Die Reduktionszeit beträgt konstant ca. 5 Stunden.

Die Bestimmung der Chlorabspaltung erfolgt in der wäßrigen Phase durch potentiometrische Titration und beträgt bei allen Ansätzen jeweils max. 4 %, bezogen auf o-Nitrochlorbenzol.

## VERGLEICHSBESPIEL ZU 1

Es wird has Beispiel 1 wiederholt, jedoch wird an Stelle des Hydroxyanthrachinon 5 g 2,3-Dichlornaphthochinon-(1,4) eingesetzt. Nach Erreichen der Azoxystufe bricht die Reaktion bei einer Reaktionstemperatur von 60°C ab und kann nur durch Steigerung der Reaktionstemperatur auf 80°C zu Ende geführt werden. Die Ausbeute beträgt 80 % d.Th., bezogen auf o-Nitrochlorbenzol. Die Reduktion dauert 6,25 Stunden.

Bei Wiedereinsatz des Palladium-Katalysators sinkt die Ausbeute gleichmäßig ab und liegt z.B. nach viermaliger Rückführung nur noch bei 76 % d.Th., während die Reduktionszeit auf 8 Studen ansteigt. Die Chlorabspaltung beträgt 8 %, bezogen auf o-Nitrochlorbenzol.

## BEISPIEL 2 Entsprechend Beispiel 1 werden

| | | |
|---|---|---|
| 630 | g | o-Nitrochlorbenzol, |
| 200 | ml | Toluol, |
| 180 | g | 25 %ige NaOH, |
| 2,5 | g | 2,6-Dihydroxyanthrachinon, |
| 2 | g | Emulgator (wie in Beispiel 1) und |
| 0,25 | g | Platin, in Form von 5 g 5 % Platin-auf-Kohlenstoff-Katalysator |

umgesetzt.

Die Ausbeute beträgt 83 % d.Th. an 2,2'-Dichlorhydrazobenzol, bezogen auf o-Nitrochlorbenzol, mit einem Schmelzpunkt von 84 bis 86°C, die Reaktionszeit beträgt 5 Stunden. Nach zehnmaliger Rückführung des Platinkatalysators sind die Ausbeuten und die Reaktionszeiten konstant. Die Chlorabspaltung beträgt max. 1,7 % d.Th., bezogen auf o-Nitrochlorbenzol.

## BEISPIEL 3

Es wird entsprechend Beispiel 1 verfahren, jedoch an Stelle des Palladium-Katalysators 0,25 g Platin, in Form von 10 g sulfitiertem 5 % Platin-auf-Kohlenstoff-Katalysator mit 50 % Wassergehalt (entsprechend DE—PS 2.105.780), eingesetzt. Die Ausbeute beträgt 83 % d.Th. an 2,2-Dichlorhydrazobenzol, bezogen auf o-Nitrochlorbenzol, mit einem Schmelzpunkt von 85 bis 86°C, die Reaktionszeit beträgt 5 Stunden. Nach zehnmaliger Rückführung des Platinkatalysators waren die Ausbeuten und die Reaktionszeiten konstant. Die Chlorabspaltung beträgt max. 0,7 % d.Th., bezogen auf o-Nitrochlorbenzol.

## Patentansprüche

1. Verfahren zur Herstellung von 2,2'-Dichlorhydrazobenzol durch katalytische Reduktion von o-Nitrochlorbenzol mit Wasserstoff in wäßriger Alkalilauge und unter Zusatz eines aromatischen, nicht wassermischbaren Lösemittels bei erhöhter Temperatur und erhöhtem Druck in Gegenwart eines Edelmetall-Katalysators, dadurch gekennzeichnet, daß als Co-Katalysator ein Anthrachinonderivat eingesetzt wird.

2. Verfahren nach Anspruch 1, dadurch gekennzeichnet, daß der Edelmetall-Katalysator ein Platin-auf-Kohle, Palladium-auf-Kohle- oder ein sulfitierter Platin-auf-Kohlenstoff-Katalysator ist.

3. Verfahren nach Anspruch 1 und 2, dadurch gekennzeichnet, daß das Anthrachinonderivat ein Hydroxyanthrachinon ist.

4. Verfahren nach Anspruch 1 und 2, dadurch gekennzeichnet, daß das Anthrachinonderivat $\beta$-Hydroxyanthrachinon oder 2,6-Dihydroxyanthrachinon ist.

5. Verfahren nach Anspruch 1 bis 4, dadurch gekennzeichnet, daß das Gewichtsverhältnis von o-Nitrochlorbenzol zum Edelmetall etwa 4000 bis 1500 : 1 beträgt.

6. Verfahren nach Anspruch 1 bis 5, dadurch gekennzeichnet, daß das Gewichtsverhältnis von o-Nitrochlorbenzol zum Anthrachinon-Derivat etwa 1 : 0,003 bis 1 : 0,008 beträgt.

## Revendications

1. Procédé de préparation du dichloro-2,2' hydrazobenzène par réduction catalytique de l'o-nitro-chlor-benzène au moyen d'hydrogène, dans une lessive alcaline aqueuse et avec addition d'un solvant aromatique non miscible à l'eau, à température élevée et sous pression élevée, en présence d'un catalyseur à base d'un métal noble, procédé caractérisé en ce qu'on utilise, comme co-catalyseur, un dérivé de l'anthraquinone.

2. Procédé selon la revendication 1, caractérisé en ce que le catalyseur à base d'un métal noble est un catalyseur constitué de platine sur charbon, un catalyseur constitué de palladium sur charbon ou un catalyseur constitué de platine sur charbon sulfité.

3. Procédé selon l'une des revendications 1 et 2, caractérisé en ce que le dérivé de l'anthraquinone est une hydroxy-anthraquinone.

4. Procédé selon l'une des revendications 1 et 2, caractérisé en ce que le dérivé de l'anthraquinone est la $\beta$-hydroxy-anthraquinone ou la dihydrox-2,6 anthraquinone.

5. Procédé selon l'une quelconque des revendications 1 à 4, caractérisé en ce que le rapport pondéral de l'o-nitro-chloro-benzène au métal noble est d'environ 4000 à 1500 : 1.

6. Procédé selon l'une quelconque des revendications 1 à 5, caractérisé en ce que le rapport pondéral de l'o-nitro-chloro-benzène au dérivé de l'anthraquinone est d'environ 1 : 0,003 à 1 : 0,008.

## Claims

1. Process for the preparation of 2,2'-dichloro-hydrazobenzene by catalytic reduction of o-nitrochlorobenzene with hydrogen in aqueous alkali metal hydroxide solution and under addition of an aromatic non-water-miscible solvent at an elevated temperature and under elevated pressure in the presence of a rare-metal catalyst, characterized by applying an anthraquinone derivative as a co-catalyst.

2. A process according to claim 1, characterized by the rare-metal catalyst being a platinum-on-carbon, a palladium-on-carbon or a sulfited platinum-on-carbon catalyst.

3. A process according to claims 1 and 2, characterized by the anthraquinone derivative being a hydroxy-anthraquinone.

4. A process according to claims 1 and 2, characterized by the anthraquinone derivative being $\beta$-hydroxy-anthraquinone or 2,6-di-hydroxy-anthraquinone.

5. A process according to claims 1 to 4, characterized by the ratio by weight of o-chloronitrobenzene to rare metal being about 4000 to 1500 : 1.

6. A process according to claims 1 to 5, characterized by the ratio by weight of o-chloronitrobenzene to the anthraquinone derivative being about 1 : 0.003 to 1 : 0.008.